# FASCICULE DE BREVET EUROPEEN

(11) **EP 1 109 525 B1**
(45) Date de publication et mention de la délivrance du brevet: **29.03.2006**
(21) Numéro de dépôt: 99936712.1
(22) Date de dépôt: 10.08.1999
(51) Int. Cl.: A61H 9/00, A61M 1/08

(54) **VENTOUSE DE MASSAGE**
MASSAGE-SAUGGLOCKE
CUPPING GLASS FOR MASSAGE

(30) Priorité: 11.08.1998 FR 9810455
(43) Date de publication de la demande: 27.06.2001
(73) Titulaire: Meslem, Jean-Claude, 34080 Montpellier (FR)
(72) Inventeur: Meslem, Jean-Claude, 34080 Montpellier (FR)
(86) Numéro de dépôt international: PCT/FR1999/001960
(87) Numéro de publication internationale: WO 2000/009067

(56) Documents cités:
- FR-A- 361 336
- FR-A- 724 281
- GB-A- 404 200
- GB-A- 1 210 746

## Description

La présente invention concerne une ventouse de massage corporel. Elle concerne plus particulièrement une ventouse de massage corporel entièrement manuelle, donc exempte de toute alimentation en énergie électrique.

Il est connu d'effectuer des massages corporels en exerçant sur l'extérieur du corps à traiter une succession d'efforts de pression, qui peuvent être accomplis manuellement, par des opérations de friction, pression, pétrissage, foulage, frappe de la main à plat ou de champ, ou effets de vibrations. Ces massages par pression ne conviennent cependant pas dans tous les cas.

On connaît également des techniques de massage procédant par succion, en particulier au moyen d'une ventouse. Pour la réalisation de massages dits du type "palper-rouler", ou massages continuels alternatifs, il a été proposé de masser les emplacements à traiter au moyen de ventouses dont le fonctionnement est dépendant d'une alimentation en énergie électrique.

On connaît également l'existence, pour les massages corporels, d'une ventouse dont le fonctionnement est entièrement manuel, c'est à dire n'ayant besoin d'aucune source d'énergie électrique.
Une telle ventouse est décrite dans le brevet FR-724281. Elle est du genre comportant, outre une ventouse rigide classique, une pompe surmontant ladite ventouse, dont elle est rendue solidaire, et comportant un corps de pompe, un axe de pompe, une tête de pompe et au moins trois valves agencées de manière appropriée et destinées respectivement à assurer :
- la décompression de la chambre de pompe,
- la décompression de la ventouse, et
- la dépression de la chambre de ventouse.

La ventouse selon l'invention se caractérise en ce que le corps de pompe pénètre à l'intérieur de la ventouse, en ce que l'échappement de l'air de la chambre de pompe s'effectue au moyen d'un conduit situé dans l'axe d'actionnement du piston de la pompe et en ce que ledit corps de pompe est relié à ladite ventouse au moyen d'une garniture assurant la liaison correspondante et l'étanchéité de celle-ci.

La ventouse selon l'invention présente entre autres comme avantage de permettre d'obtenir une dépression contrôlée ou maîtrisée. En effet, la valve assurant la décompression de la ventouse permet de faire pénétrer une quantité dosée d'air dans la cloche, si on le souhaite et/ou si l'on constate qu'une dépression trop forte a été produite par la mises en oeuvre des autres moyens.

L'invention est décrite ci-après plus en détail en référence aux dessins annexés qui en représentent des formes de réalisation non limitatives et dans lesquels :
- la Fig- 1 est une représentation en coupe longitudinale schématique du dispositif à ventouse selon l'invention, en position basse;

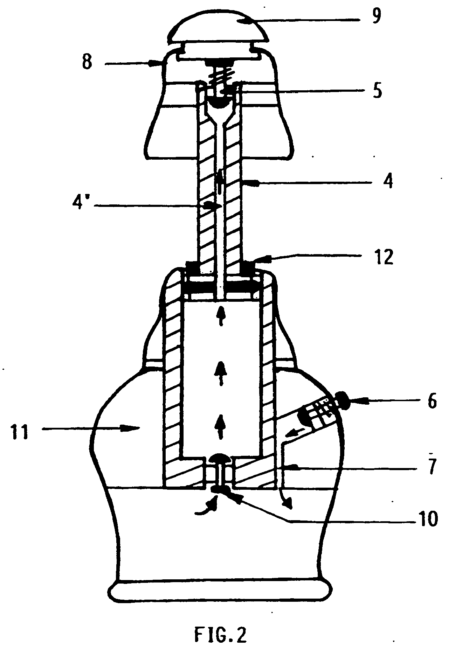

- la Fig. 2 est une représentation en coupe longitudinale schématique du dispositif à ventouse selon l'invention, en position relevée.

Une ventouse conforme à l'invention comporte ainsi une ventouse (1) proprement dite, en matériau rigide tel que, par exemple, du verre, surmontée par une pompe fixée sur la ventouse au moyen d'une garniture de pompe (2) et dont le corps de pompe (3) pénètre à l'intérieur du volume de la ventouse. La partie externe de la pompe comporte un axe (4) comportant un conduit d'échappement d'air (4') et se terminant à son extrémité supérieure par une valve (5) de décompression de la chambre de pompe. Une valve (6) de décompression de la ventouse, semblable quant à sa structure et à son agencement à la valve (5), permet d'agir sur un conduit secondaire (7) pour une entrée d'air destinée à piloter la décompression de la chambre de ventouse.

Ladite valve (5) est elle-même enfermée dans un pommeau (8), et un bouton-poussoir (9) ou un organe similaire surmonte la valve (5) pour rendre plus aisé son actionnement Ladite valve (5) est maintenue en position fermée par un ressort de rappel et une pression sur le bouton-poussoir (9) a pour effet, si elle est suffisante, d'ouvrir cette valve (5).

La valve (6) susmentionnée comporte de préférence un pommeau et/ou un bouton-poussoir actionnant un ressort de rappel semblables à ceux dont est munie la valve (5). Là encore, la valve est maintenue en position fermée grâce à l'action du ressort de rappel, à laquelle il suffit de s'opposer par une pression suffisante sur le bouton-poussoir pour amener ladite valve (6) en position ouverte temporaire.

Le corps de pompe (3) comporte à son extrémité située à l'intérieur de la ventouse, et donc à l'opposé de la valve (5) surmontant l'axe de pompe (4), une valve (10) de dépression de la chambre de ventouse. Cette valve (10) est de préférence un clapet libre.

Selon une forme de réalisation particulièrement préférée, la ventouse comporte en outre du silicone pigmenté de remplissage (11), qui permet de donner au volume de la ventouse un aspect coloré, dont la teinte peut être choisie à convenance, par exemple en correspondance avec des applications différentes et conformes à des codes de couleurs connus ou définis.

De préférence, la fixation de l'axe de pompe (4) sur le corps de pompe (3) est améliorée au moyen d'un guide-axe (12), par exemple en caoutchouc.

Pour utiliser la ventouse selon l'invention à des fins de massage, on procède comme suit :

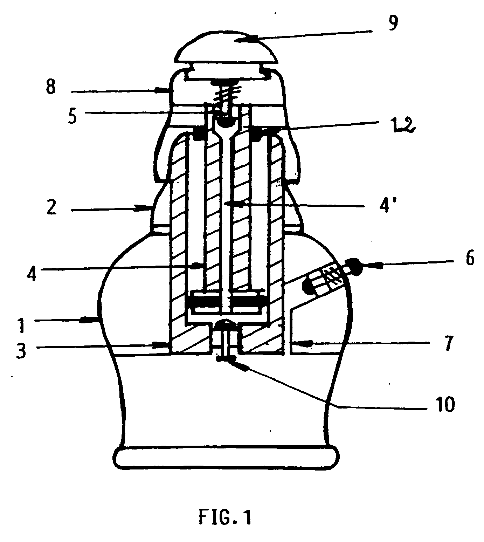

On pose la ventouse de verre combinée à une pompe selon l'invention, en position basse (voir Fig. 1) sur le corps de la personne à masser, tout en appuyant légèrement, de façon à assurer une étanchéité convenable entre le verre de la ventouse et la peau.

Tout en tenant d'une main la ventouse de façon à maintenir sa surface de verre contre la peau, on tire avec l'autre main sur le pommeau (8) de l'axe (4), jusqu'à ce que soit atteinte la position relevée extrême ou position haute maximale (voir Fig. 2). L'air contenu dans la chambre de ventouse passe ainsi par la valve à clapet libre (10) et il se forme une dépression dans la ventouse. On constate une légère aspiration de la peau à l'intérieur de la ventouse.

Si cette dépression créée dans la ventouse n'est pas suffisante, il suffit de ramener l'axe de pompe (4) en position basse, en ayant soin d'appuyer sur le bouton-poussoir (9), et celui-ci permet d'évacuer l'air contenu dans la pompe, à travers le conduit d'axe (4') et de la valve (5), qui est de préférence une valve à ressort En procédant ainsi, on ne modifie pas la dépression précédemment obtenue dans la ventouse.

Lorsque l'aspiration obtenue est estimée correcte, on peut procéder au massage selon les techniques classiques connues.

Si la dépression ainsi créée est trop faible, on peut recommencer l'opération d'aspiration autant de fois qu'il le faut, en pratique une ou deux fois seulement, en opérant comme décrit ci-dessus.

Si la dépression créée dans la ventouse est trop forte, il suffit d'agir sur le bouton-poussoir commandant la valve (6) du conduit secondaire (7) de décompression. De l'air pénètre alors dans la ventouse et cela permet de contrôler ou régler la dépression dans celle-ci.

L'entraînement de l'air se fait dans le sens et les directions indiqués par les flèches sur la figure 2.

C'est en appuyant de même sur le bouton-poussoir du conduit secondaire ou latéral (7) que l'on dégage la ventouse en fin de massage.

Avant de placer ce dispositif comme on vient de l'indiquer, il est recommandé d'appliquer une huile de massage sur la peau du corps à masser.

La ventouse selon l'invention permet d'effectuer des massages sur les parties du corps humain ou animal qui doivent être ainsi traitées et procure à la peau une action de succion bénéfique aux endroits traités. Elle peut en particulier être utilisée pour le massage de nerfs sensibles et/ou malades, pour le massage des zones dites de Head, le massage des tissus conjonctifs et le massage des cicatrices adhérentes.

Dans toutes ces applications la sécurité d'emploi est assurée et est conforme aux exigences des régiementations en vigueur en la matière.

Bien entendu, les formes de réalisation de l'invention qui ont été décrites ci-avant ont été données à titre purement indicatif et nullement limitatif.

## Revendications

1. Ventouse pour le massage du genre comportant :
- une ventouse rigide (1);
- une pompe, solidaire de ladite ventouse, constituée d'un corps (3), d'un piston et d'un axe d'actionnement (4);
- une valve (5) assurant la décompression de la chambre de pompe;
- une valve (6) assurant la décompression de la ventouse;
- une valve (10) assurant la dépression de ladite chambre;
**caractérisée en ce que** le corps de pompe (3) pénètre à l'intérieur de la ventouse (1); **en ce que** l'échappement de l'air de la chambre de pompe s'effectue au moyen d'un conduit (4') situé dans l'axe (4) d'actionnement du piston de la pompe et **en ce que** ledit corps de pompe (3) est relié à ladite ventouse au moyen d'une garniture (2) assurant la liaison correspondante et l'étanchéité de celle-ci.

2. Ventouse, selon la revendication 1, **caractérisée en ce que** la valve (5) comporte un bouton-poussoir (9) d'actionnement manuel et un ressort de rappel destiné à la maintenir en position fermée.

3. Ventouse, selon la revendication 1, **caractérisée en ce que** la valve (6) comporte un bouton-poussoir d'actionnement manuel et un ressort de rappel destiné à la maintenir en position fermée.

4. Ventouse, selon la revendication 1, **caractérisée en ce que** la valve (10) est constituée d'un clapet libre.

5. Ventouse, selon la revendication 1, **caractérisée en ce que** la ventouse (1) comporte du silicone pigmenté de remplissage (11).

## Patentansprüche

1. Massageventuse der Art bestehend aus:
- einer steifen Ventuse (1),
- einer zur besagten Ventuse gehörigen Pumpe bestehend aus einem Körper (3), einem Kolben und einer Betätigungsachse (4),
- einem Ventil (5) für die Dekomprimierung der Pumpenkammer,
- einem Ventil (6) für die Dekomprimierung der Ventuse
- einem Ventil (10) für die Drucksenkung in der besagten Kammer,
**dadurch charakterisiert**, dass der Pumpenkörper (3) in das Innere der Ventuse (1) hineinragt, wie auch dadurch, dass das Entweichen der Luft aus der Pumpenkammer mittels einer Leitung (4') erfolgt, die in der Betätigungsachse (4) des Pumpenkolbens liegt, und dass der besagte Pumpenkörper (3) mit der besagten Ventuse mittels einer Dichtung (2) verbunden ist, welche die entsprechende Verbindung und Dichtigkeit dieser sicher stellt.

2. Einer Ventuse nach Anspruch 1, **dadurch charakterisiert**, dass das Ventil (5) einen Druckknopf (9) zur manuellen Betätigung und eine Rückholfeder hat, welche dazu bestimmt ist, das Ventil in geschlossener Stellung zu halten.

3. Einer Ventuse nach Anspruch 1, charakterisiert dadurch, dass das Ventil (6) einen Druckknopf zur manuellen Betätigung und eine Rückholfeder hat, welche dazu bestimmt ist, das Ventil in geschlossener Stellung zu halten.

4. Einer Ventuse nach Anspruch 1, **dadurch charakterisiert**, dass das Ventil (10) aus einer Entlüftungsklappe besteht.

5. Einer Ventuse nach Anspruch 1, **dadurch charakterisiert**, dass die Ventuse (1) eine Einlage (11) aus pigmentiertem Silikon enthält.

## Claims

1. A suction cap for massaging including:
- a rigid suction cap (1);
- a pump, fixed to said suction cap, made up of a structure (3), a piston and a driving axle (4);
- a valve (5) guaranteeing decompression of the pump chamber;
- a valve (6) guaranteeing decompression of the suction pad;
- a valve (10) guaranteeing depression of the above-mentioned chamber
**Characterised in that** the structure of the pump (3) penetrates inside the suction pad (1); that the air exhaust valve of the chamber of the pump is carried out through a conduit (4') inserted in the axle (4) that activates the piston of the pump and **in that** the above mentioned structure of the pump (3) is connected with the above mentioned suction pad by a washer (2) guaranteeing corresponding connection and relative tightness.

2. A suction cap, as per claim 1, **characterised in that** the valve (5) includes a button-switch (9) for manual activation and a recall spring to keep it in a firm position.

3. A suction cap, as per claim 1, **characterised in that** the valve (6) includes a button-switch for manual activation and a recall spring to keep it in a firm position.

4. A suction cap, as per claim 1, **characterised in that** the valve (10) is made up of a free valve.

5. A suction cap, as per claim 1, **characterised in that** the suction cap (1) is made up of a pigmented filling silicone.
